# EUROPÄISCHE PATENTSCHRIFT

(11) **EP 1 392 375 B1**
(45) Veröffentlichungstag und Bekanntmachung des Hinweises auf die Patenterteilung: **10.09.2008**
(21) Anmeldenummer: 02760166.5
(22) Anmeldetag: 15.05.2002
(51) Int. Cl.: A61M 1/02

(54) **ZUSAMMENSETZUNG UND VERWENDUNG VON SUBSTANZEN ZUR STABILISIERUNG VON SCHWEFELHALTIGEN AMINOSÄUREN UND/ODER ZUR HEMMUNG DER FORTLAUFENDEN BILDUNG VON SCHWEFELHATIGEN AMINOSÄUREN IN ENTNOMMENEM BLUT**
COMPOSITION AND USE OF SUBSTANCES FOR STABILISING AMINO ACIDS CONTAINING SULPHUR
COMPOSITION ET UTILISATION DE SUBSTANCES POUR STABILISER DES ACIDES AMINES SULFUREUX

(30) Priorität: 21.05.2001 DE 10124820
(43) Veröffentlichungstag der Anmeldung: 03.03.2004
(73) Patentinhaber: WIELAND, Heinrich, 79271 St. Peter (DE)
(72) Erfinder: WIELAND, Heinrich, 79271 St. Peter (DE); BISSE, Emanuel, 79211 Denzlingen (DE)
(74) Vertreter: Hofer, Dorothea
(86) Internationale Anmeldenummer: PCT/EP2002/005368
(87) Internationale Veröffentlichungsnummer: WO 2002/094248

(56) Entgegenhaltungen:
- EP-A- 1 085 083
- WO-A-00/13691
- WO-A-00/78311
- WO-A-01/81920
- US-A- 4 968 690
- US-A- 5 872 104
- NAUCK M; BISSE E; NAUCK M; WIELAND H: "Pre-Analytical conditions affecting the determination of the plasma homocysteine concentration" CLINICAL CHEMISTRY AND LABORATORY MEDICINE, Bd. 39, Nr. 8, August 2001 (2001-08), Seiten 675-680, XP009002815
- AL-KHAFAJI; BOWRON A; DAY A P; SCOTT J; STANSBIE D: "Stabilization of blood homocysteine by 3-deazaadenosine" ANN CLIN BIOCHEM, Nr. 35, 1998, Seiten 780-782, XP002225386 in der Anmeldung erwähnt
- SIMING LIU ET AL: "RATIONAL APPROACHES TO THE DESIGN OF ANTIVIRAL AGENTS BASED ON S-ADENOSYL-L-HOMOCYSTEINE HYDROLASE AS A MOLECULAR TARGET" ANTIVIRAL RESEARCH, ELSEVIER SCIENCE BV., AMSTERDAM, NL, Bd. 19, Nr. 3, 1. September 1992 (1992-09-01), Seiten 247-265, XP000601270 ISSN: 0166-3542 in der Anmeldung erwähnt
- WILLEMS H; BOS G; GERRITS W: "Acidic Citrate stabilizes blood samples for assay of total homocystein" CLINICAL CHEMISTRY, Bd. 44, 1998, Seiten 342-345, XP002225387
- SASO Y; CONNER E M; TEEGARDEN B R; ET AL: "S-Adenosyl-L-homocysteine Hydrolase Inhibitor mediates immunosuppressive effects in vivo" J. PHARMACOLOGY AND EXPERIMENTAL THERAPEUTICS, Bd. 296, Nr. 1, 2001, Seiten 106-112, XP002225857
- PATENT ABSTRACTS OF JAPAN & JP 56 051414 A (TOYO JOZO KK), 9. Mai 1981 (1981-05-09)

## Beschreibung

Die Erfindung bezieht sich auf eine Zusammensetzung zur Stabilisierung von schwefelhaltigen Aminosäuren in entnommenem Blut, auf die Verwendung geeigneter Substanzen und Zusammensetzungen hierfür sowie gegebenenfalls zur Bestimmung von schwefelhaltigen Aminosäuren im Blut, auf ein Verfahren zu diesen Zwecken sowie auf eine für dieses Verfahren geeigneter Weise eingesetzte Blutsammelvorrichtung.

In zahlreichen prospektiven Studien und Fallstudien konnte in den letzten Jahren gezeigt werden, dass Homocystein im Blutplasma ein unabhängiger reversibler Risikofaktor für kardiovaskuläre, atherosklerotische, neurologische und geriatrische Erkrankungen ist. Eine mäßig erhöhte Plasmakonzentration des Homocysteins findet man bei etwa 8 % der allgemeinen Bevölkerung und bei 20 bis 40 % der Patienten mit koronaren oder peripheren Gefäßerkrankungen. Eine deutliche Erhöhung des Homocysteins findet man bei Patienten mit Homocysteinurie, die auf angeborenen Stoffwechseldefekten beruht. Der Zusammenhang zwischen der Plasmakonzentration des Homocysteins und kardiovaskulären Erkrankungen ist noch nicht eindeutig geklärt. Der wesentliche Angriffspunkt scheint die Endothelzelle zu sein. Homocystein hemmt das Wachstum, vermindert die Aktivität der Glutathionperoxidase, führt zu Veränderungen der Genexpression, zu einer verminderten Expression des Thrombomodulins und zu einer verminderten Bindung des Gewebsplasminogenaktivators sowie zu einer verminderten Sekretion des von-Willebrand-Faktors. Darüber hinaus kann Homocystein die Proliferation vaskulärer glatter Muskelzellen induzieren. Als weiterer schädigender Mechanismus wird die Bildung freier Radikale durch Homocystein in Gegenwart von Eisen- oder Kupferionen angeführt, die zu einer oxidativen Veränderung der lowdensity Lipoproteine führen kann.

Es ist nicht klar, ob Homocystein selbst für das gesteigerte kardiovaskuläre Risiko verantwortlich ist, denn die erwähnten Mechanismen gründen sich überwiegend auf Daten, die anhand von Tierversuchen und Zellkulturen gewonnen wurden. Bei solchen Versuchen wurden recht hohe Homocysteinkonzentrationen verwendet (1 bis 10 mmol/l). Darüber hinaus kommt das reduzierte Homocystein, die Form, die bei den meisten in-vitro-Studien verwendet wurde, in Konzentrationen von unter 1 µmol/l vor.

Ein weiteres Problem klinischer Studien besteht bisher darin, dass der gemessene Plasma-Homocysteinspiegel nur dann als verlässlich gelten kann, wenn die Blutzellen schnellstmöglich nach der Blutabnahme abzentrifugiert werden (< 30 Minuten), damit das Serum so schnell wie möglich gewonnen werden kann. Dieses Verfahren ist aber sowohl für niedergelassene Ärzte als auch für Kliniker nicht besonders praktikabel.

Diese schnellstmögliche Gewinnung des Blutserums ist deshalb erforderlich, da die Blutzellen noch nach der Blutabnahme Homocystein weiter produzieren (bis zu 40 % Erhöhung der Konzentration im Plasma nach 1 Stunde). Dies ist vermutlich darauf zurückzuführen, dass es innerhalb der Blutzellen zu andauernden Methylübertragungen durch S-Adenosylmethionin kommt. Die Bildung von S-Adenosylmethionin hält an, solange das Serum oder Plasma den Erythrozyten noch Methionin zur Verfügung stellen kann. Wenn S-Adenosylmethionin die Methylgruppe gespendet hat, entsteht daraus S-Adenosylhomocystein, welches zu Homocystein weiter hydrolysiert wird. Damit haben sowohl der Methioninspiegel des Serums als auch die Zeitdauer bis zur Gewinnung des Serums einen erheblichen Einfluss auf den Homocysteinspiegel im Blutserum. Dies mag ein wesentlicher Grund für wenig eindeutige Ergebnisse bezüglich der Aussagekraft des Homocysteinspiegels beim Menschen für koronare und periphere Gefäßkrankheiten sein. Damit Homocystein richtig und reproduzierbar bestimmt werden kann, müssen die präanalytischen Anforderungen für eine verlässliche Bestimmung des Homocysteins erfüllt sein, d.h. es sollte die fortlaufende Homocysteinbildung unterbrochen sein.

Frühere Untersuchungen auf diesem Gebiet stellten F. Al-Khafaji, A. Bowron, A. Day, J. Scott und D. Stansbie in einem Vortrag auf dem Kongress der Association of Clinical Biochemists (ACB) (Proceedings ACB National Meeting 1998, Glasgow, 11. bis 15. Mai 1998, p. 21) vor. Sie berichteten, dass mittels NaF die Hemmung des Methioninabbaus zu 2-Adenosylmethionin gelang und deshalb in den Blutproben kein Anstieg von Homocystein zu beobachten war. Mit dem Inhibitor NaF kam es im Vergleich zu dem anfänglichen Wert (Zeitpunkt der Blutentnahme) zu einer Absenkung des Homocysteinspiegels im Blut, was bei einer verlässlichen Präanalytik nicht erwünscht ist.

Weiterhin untersuchten die Autoren die Wirksamkeit von 3-Deazaadenosin, einem bekannten Inhibitor für die S-Adenosylhomocystein-Hydrolase, auf die Stabilisierung des Homocysteinspiegels. Sie berichteten, dass mit diesem herkömmlichen Inhibitor die Umsetzung von S-Adenosylhomocystein zu Homocystein wirksam bis zu 72 Stunden gehemmt werden kann.

Detailliertere Untersuchungsergebnisse veröffentlichte diese Forschergruppe in einem Kurzbericht (F. Al-Khafaji, A. Bowron, A. Day, J. Scott und D. Stansbie, Ann Clin. Biochem., 1998, 35, 780-782). Es zeigte sich, dass durch Zusatz des herkömmlichen reversiblen und kompetitiven Inhibitors für das Enzym S-Adenosylhomocystein-Hydrolase, nämlich 3-Deazaadenosin, kein Anstieg des Homocysteinspiegels im Blut über 24 Stunden beobachtet werden konnte. Auch der im weiteren Verlauf erfolgte Anstieg des Homocysteinspiegels um ungefähr 10 % bei einem Zeitpunkt von 72 Stunden nach der Blutabnahme lag weit unter den Vergleichswerten mit Probenröhrchen, die nur das herkömmlicher Weise verwendete Anti-Koagulationsmittel Ethylendiamintetraessigsäure (EDTA) enthielten.

Ein Problem, das bei der Verwendung von 3-Deazaadenosin auftritt, besteht darin, dass merkliche Schwankungen der gemessenen Werte auftreten, die bei Homocystein im Bereich von bis zu 2 µmol/l in 60 Stunden, sowie bei Cystein im Bereich von 60 µmol/l in 60 Stunden liegen. Zudem kann der Gehalt an Cysteinylglycin und Glutathion nicht festgestellt werden, da 3-Deazaadenosin deren Bildung nicht hemmt. Ein weiteres Problem besteht darin, dass 3-Deazaadenosin bei Raumtemperatur instabil ist und nur gekühlt (+4°C) lagerfähig ist, was auch seine Transportfähigkeit einschränkt. In der diagnostische Medizin ist eine einfache Handhabung der Reagenzien aber von großer Bedeutung, da beispielsweise entnommene Blutproben ohne größeren Aufwand, wie beispielsweise Kühlung, über einen längeren Zeitraum in unveränderter Form vorliegen und transportfähig sein müssen.

Das P-Dokument M. Nauck et al., Clin. Chem. Lab. Med. 2001, 39(8) S. 675-680, beschreibt mögliche Stabilisierungssubstanzen, u.a. Natriumfluorid und 3-Deazaadenosin, zur Stabilisierung von Homocystein im Blut.

Die WO 01/81920 beschreibt eine vergleichende Phänotyp-Analyse zur Untersuchung von biologisch aktiven Verbindungen wie antimikrobiellen Wirkstoffen. In einem Testsatz mit 95 verschiedenen Nahrungszusatzstoffen zu einer Zellsuspension wurden u.a. 2'-Dcoxyadenosin oder 4-Aminoimidazol-4 (5) -carboxamid zugesetzt.

Die WO 00/13691 beschreibt die Verwendung von 3-Deazaadenosin und dessen Analoga zur Herstellung eines Medikaments für Gefäßerkrankungen und Organabstoßung.

Aufgabe der vorliegenden Erfindung ist es die Präanalytik für die Untersuchung der schwefelhaltigen Aminosäuren zu verbessern. Insbesondere sollen die im Stand der Technik angesprochenen Probleme der Schwankung des Homocystein-und Cysteinspiegels, der Messung des Gehalts an Cysteinylglycin und Glutathion, der einfachen Handhabung, Lagerung und Transportfähigkeit gelöst werden.

Diese Aufgabe wird gelöst durch eine Zusammensetzung gemäß Anspruch 1, durch Verwendungen gemäß Anspruch 11 und 17, durch ein Verfahren gemäß Anspruch 24, und durch eine Blutsammelvorrichtung gemaß Anspruch 39. Bevorzugte Ausführungsformen der Erfindung sind in den Unteransprüchen zu den vorstehend genannten Ansprüchen angegeben.

Die vorstehend genannten und weitere Lösungen der erfindungsgemäßen Aufgabe mit ihren Wirkungen und Vorteilen ergeben sich aus der nachstehenden, näheren Beschreibung der Erfindung und aus den bevorzugten Ausführungsformen.

Als nächstes werden die Figuren detaillierter erklärt:
Fig. 1 veranschaulicht den Anstieg der Konzentration an Homocystein (HCY) im Vollblut, das in Röhrchen gesammelt wurde, die Ethylendiamintetraessigsäure (EDTA) und Citronensäure enthielten. Die Proben wurden bei Raumtemperatur aufbewahrt.
Fig. 2 veranschaulicht den Anetieg der HCY-konzentration im Vollblut, das in Röhrchen gesammelt wurde, die EDTA enthielten. Die Proben wurden bei Raumtemperatur aufbewahrt.
Fig. 3 veranschaulicht die Stabilisierungswirkung von Citronensäure + A (eine Mischung aus EDTA, 5(4)-Aminoimidazol-4(5)-carboxamid und 2'-Deoxyadenosin) und von 3-Deazaadenosin auf die HCY-Konzentration. Die Proben wurden in EDTA-Röhrchen entnommen, die Citronensäure + A beziehungsweise 3-Deazaadenosin enthielten. Sie wurden bei Raumtemperatur aufbewahrt.
Fig. 4 veranschaulicht die Stabilisierungswirkung von Citronensäure + 3-Deazaadenosin (3-DEAD) und von 3-Deazaadenosin auf die HCY-Konzentration. Die Proben wurden in EDTA-Röhrchen entnommen, die Citronensäure + 3-DEAD beziehungsweise 3-DEAD enthielten. Sie wurden bei Raumtemperatur aufbewahrt.
Fig. 5 veranschaulicht die Stabilisierungswirkung von Citronensäure + A und von 3-Deazaadenosin auf die Konzentration von Cystein (CYS). Die Proben wurden in EDTA-Röhrchen entnommen, die Citronensäure + A beziehungsweise 3-Deazaadenosin enthielten. Sie wurden bei Raumtemperatur aufbewahrt.
Fig. 6 veranschaulicht die Stabilisierungswirkung von Citronensäure + A und von 3-Deazaadenosin auf die Konzentration von Cysteinylglycin (CYSGLY). Die Proben wurden in EDTA-Röhrchen entnommen, die Citronensäure + A beziehungsweise 3-Deazaadenosin enthielten. Sie wurden bei Raumtemperatur aufbewahrt.
Fig. 7 veranschaulicht die Stabilisierungswirkung von Citronensäure + A und von 3-Deazaadenosin auf die Konzentration von Glutathion (GLU). Die Proben wurden in EDTA-Röhrchen entnommen, die Citronensäure + A beziehungsweise 3-Deazaadenosin enthielten. Sie wurden bei Raumtemperatur aufbewahrt.
Fig. 8 veranschaulicht die Stabilisierungswirkung von 3-Deazaneplanocin A (3DA-NeplA) auf-die HCY-Konzentration. Die Proben wurden in EDTA-Röhrchen und Na-Heparin-Röhrchen entnommen, die jeweils 3DA-NeplA enthielten und bei Raumtemperatur aufbewahrt wurden.

Nachfolgend wird unter Bezugnahme auf die beigefügten graphischen Darstellungen die Wirkungen und die Vorteile der Erfindung sowie bevorzugte Ausführungsformen der vorliegenden Erfindung beschrieben.

Der erfindungsgemäß eingesetzte, wenigstens eine Inhibitor ist ein solcher, der eines der an der metabolischen Homocysteinbildung beteiligten Enzyme inhibiert. Gegebenenfalls kann der gleiche Inhibitor auch ein Inhibitor für ein weiteres an der Homocysteinbildung beteiligtes Enzym sein. Als Angriffspunkte für die Inhibierung gelten bevorzugt eine S-Adenosylhomocystein-Hydrolase, eine Methyltransferase, bei der S-Adenosylmethionin als Methyldonor dient, oder jedes andere an der Homocysteinbildung oder am Homocysteinabbau beteiligte Enzym.

Der für alle Aspekte der Erfindung einsetzbare, wenigstens eine Inhibitor hat vorzugsweise eine Struktur, dass er nicht-ionisch ist und/oder so hydrophob ist, dass er zur Permeation einer Erythrocytenmembran in der Lage ist. Der Inhibitor unterscheidet sich damit von dem Reagens NaF bzw. von indirekten, über eine Calciumionenbindung erfolgenden Stabilisierungen mit Chelatbildnern wie EDTA oder Citrat. Mit dieser bevorzugten Inhibitorstruktur ist eine Lyse der Blutzellen nicht erforderlich. Da ein Vermeiden der Blutzelllyse zudem von Vorteil ist, werden erfindungsgemäß vorzugsweise keine Agenzien zur Lyse von Blutzellen angewandt.

Gemäß dem ersten Aspekt der Erfindung, bei dem der Inhibitor auch ohne Säure die gewünschten Wirkungen erzielt, hat der eingesetzte Inhibitor nicht lediglich eine kompetitiv hemmende Wirkung auf eines der genannten, an der Homocysteinbildung beteiligtes Enzyme Somit unterscheidet sich dieser Ansatz signifikant von dem herkömmlich verwendeten, kompetitiv hemmenden 3-Deazaadenosin, bei dem noch relativ große Schwankungen in der Stabilisierung zeigten. Zu der Gruppe der nicht lediglich kompetitiv hemmenden Inhibitoren gehören vorzugsweise irreversible Inhibitoren und insbesondere sogenannte Suizid-Inhibitoren. Es ist von Vorteil, wenn das Enzym S-Adenosylhomocystein--Hydrolase mit einem entsprechenden Inhibitor gehemmt wird. Als besonders im Sinne des ersten Aspekts der Erfindung effektive Inhibitoren sind vor allem solche einzusetzen, die zur Inaktivierung der S-Adenosylhomocysteinhydrolase unter Erschöpfung des Cofaktors NAD⁺ führen. Beispiele für vorteilhafte Inhibitoren schließen Neplanocin A, 3-Deazaneplanocin A, 9- (*trans*-2'-, *trans*-3'-dihydroxycylopent-4'-enyl)-adenosin (DHCeA), 3-Deaza-DHCeA, 9- (*trans-*2'-, *trans*-3' -dihydroxycylopentanyl) - adenosin (DHCaA), 3-Deza-DHCaA, β-4' -Methyl-DHCaA, β-4'-Vinyl-DHCaA, (Z- oder E) -4',5' -Didehydro-5'-deoxy-5'-fluoro-adenosin (Z-DDFA oder E-DDFA) und 5'-Deoxy-5'-difluoro-adenosin (DFA) sowie den inhibitorischen Derivaten oder Analoga der genannten Verbindungen ein. Diese Inhibitoren werden als sogenannte S-Adenosylhomocysteinhydrolase-Inhibitoren der zweiten Generation als antivirale Mittel von S.Liu, M.S.Wolfe und R.T.Borchardt zusammenfassend beschrieben in Antiviral Research 19 (1992), 247-365. Der Grund für den signifikanten Unterschied zur lediglich kompetitiven Hemmung wird darin vermutet, dass es mit den genannten Inhibitoren zu einer Verarmung an verfügbarem cofaktor NAD⁺ unter Oxidierung des eingesetzten Inhibitors zur 3'-Ketoform und ggf. zu einer stabilen Bindung der oxidierten 3'-Ketoform im Enzym kommt.

Zusätzliche vorteilhafte Wirkungen können durch weitere Zusatzstoffe erzielt werden. So wurde im Rahmen des zweiten Aspekts der Erfindung überraschenderweise festgestellt, dass durch eine Zusammensetzung gemäß dem zweiten Aspekt der Erfindung bei annähernd gleicher wirkung eine kostengünstige Lösung der erfindungsgemäßen Aufgabe erreicht werden kann. Durch eine Zusammensetzung, die neben wenigstens einem Inhibitor für eines der genannten, an der Homocysteinbildung beteiligtes Enzyme zusätzlich noch wenigstens eine Säure umfasst, kann eine verbesserte Stabilisierung von schwefelhaltigen Aminosäuren und/oder eine verbesserte Hemmung der Bildung von schwefelhaltigen Aminosäuren im Blut erreicht werden. In diesem Fall ist aufgrund des erzielten synergistischen Effekts mithilfe der Säure sogar ein herkömmlicher kompetitiver Inhibitor wie 3-Deazaadenosin einseztbar.

Die in diesen erfindungsgemäßen Zusammensetzungen verwendeten, mit einer Säure kombinierten Inhibitoren für das wenigstens eine an der Homocysteinbildung beteiligte Enzym können aus der folgenden Gruppe ausgewählt werden: 2'-Deoxyadenosin (DEOA), 5(4)-Aminoimidazol-4(5)-carboxamid (5(4)-AMCA), 9(S)-(2,3-Dihydroxypropyl)-adenosin ((S)-DHPA), (R,S)-3-Adenin-9-yl-2-hydroxypropansäure ((R,S)-AHPA), Adenin-dialdehyd, 3-Deazaadenosin, 5-Deoxy-5-methyl-thioadenosin, 5-Deoxyadenosin (Aristeromycin), Neplanocin A, 3-Deazaneplanocin A, 9-(*trans*-2'-, *trans*-3'-dihydroxycylopent-4'-enyl)-adenosin (DHCeA), 3-Deaza-DHCeA, 9-(*trans*-2'-,*trans*-3'-dihydroxycylopentanyl)-adenosin (DHCaA), 3-Deza-DHCaA, β-4'-Methyl-DHCaA, β-4'-Vinyl-DHCaA, Z- oder E-4',5'-Didehydro-5'-deoxy-5'-fluoro-adenosin (Z-DDFA oder E-DDFA) und 5'-Deoxy-5'-difluoro-adenosin (DFA) sowie den inhibitorischen Derivaten oder Analoga der genannten Verbindungen besteht. Bezüglich der an sich als Antivirusmittel bekannten Substanzen sei wiederum auf den o.g. Übersichtsartikel von S.Liu, M.S.Wolfe und R.T.Borchardt in Antiviral Research 19 (1992), 247-265 (mit weiteren Nachweisen) verwiesen.

Als für den zweiten Aspekt der Erfindung besonders geeignete Inhibitoren sind 3-Deazadenosin, 5-Deoxy-5-methyl-thioadenosin und dessen inhibitorischen Derivate sowie die im Zusammenhang mit dem ersten Aspekt der Erfindung beschriebenen, insbesondere die irreversiblen Inhibitoren der besagten Enzyme wie Neplanocin A und 3-Deazaneplanocin A und den inhibitorischen Derivaten der genannten Verbindungen zu nennen.

Die in der von der Art des Inhibitors unabhängigen Zusammensetzung eingesetzten Säuren gemäß dem zweiten Aspekt der Erfindung sind vorzugsweise organische Säuren und vor allem solche, die keine Lyse der Erythrozyten hervorrufen. Die Säure wird bevorzugt allein oder in Kombination aus folgender Gruppe ausgewählt: Ascorbinsäure, Citronensäure, Citramalsäure, Citraconsäure, Fumarsäure, Milchsäure, Oxalsäure, Weinsäure und andere organische Mono-, Di-, und höherwertige Säuren. Das Auftreten einer Lyse der Erythrozyten würde die Analytik auf Grund eines dadurch hervorgerufenen Verdünnungseffekts verfälschen. Die am meisten bevorzugte Säure ist Citronensäure.

Die Säure kann grundsätzlich in jeder beliebigen Form der Zusammensetzung hinzugefügt sein. Die Erfinder fanden aber überraschend, dass die Stabilisierungswirkung der schwefelhaltigen Aminosäuren begünstigt ist, wenn die Säure in fester Form und bevorzugt pulverisiert in der Zusammensetzung vorliegt. Eine besonders günstige Wirkung hatte die in der erfindungsgemäßen Zusammensetzung eingesetzte Säure, wenn sie in lyophylisierter Form vorlag.

Mit den erfindungsgemäßen Zusammensetzungen kann nicht nur der Gehalt an Homocystein und Cystein im entnommenen Blut mit geringeren Schwankungen als in den herkömmlichen Stabilisierungssystemen, sondern auch der Gehalt an Cysteinylglycin und Glutathion konstant bis zu 54 Stunden, günstigerweise bis zu 72 Stunden und teilweise sogar bis zu über 100 Stunden stabilisiert werden (z.B. eine Stabilisierung des Homocysteins von mindestens 143 Stunden bei Raumtemperatur mit 3-Deazaneplanocin A). Zusätzlich sind die mit der erfindungsgemäßen Zusammensetzung versetzten Blutproben über einen längeren Zeitraum bei Raumtemperatur lagerfähig und transportierbar.

Eine Kombination aus einem oder mehreren kompetitiven Inhibitor(en) und/oder einem oder mehreren irreversiblen Inhibitor(en) für die vorstehend erwähnten Enzyme kann für die Stabilisierung des Homocysteinspiegels im Vollblut durch die sich günstig ergänzende Wirkung von Vorteil sein. Dies ist dann besonders wirksam, wenn mindestens ein Inhibitor der Methyltransferase und/oder mindestens ein kompetitiver oder nicht kompetitiver Inhibitor der S-Adenosylhomocystein-Hydrolase vorliegt bzw. vorliegen. Gute Ergebnisse wurden insbesondere durch eine Kombination aus den beiden Inhibitoren 2'-Deoxyadenosin und 5(4)-Aminoimidazol-4(5)-carboxamid erzielt.

Außerdem kann die erfindungsgemäße Zusammensetzung noch jeden weiteren Zusatzstoff enthalten, der in herkömmlichen Zusammensetzungen für die Präanalytik von Blut eingesetzt wird.

Für die Zusammensetzung der Erfindung ist beispielsweise die Einstellung des pH-Wertes auf einen physiologisch günstigen Wert nützlich. Deshalb können der erfindungsgemäßen Zusammensetzung unter anderem Puffer, zum Beispiel in fester, flüssiger oder lyophylisierter Form, zugegeben werden. Der Anteil an Puffer wird so bestimmt, dass dieser nach der Vermischung der Substanz genügend Pufferwirkung entfaltet, um den pH-Wert auf einen Bereich von etwa pH 5 bis etwa pH 9, bevorzugt etwa pH 6 bis etwa pH 8 und insbesondere etwa pH 6,5 bis etwa pH 7,5 einzustellen. Als günstig für die Stabilisierung von schwefelhaltigen Aminosäuren hat sich die Verwendung eines herkömmlichen Phosphatpuffers erwiesen und bevorzugt die eines Phosphatpuffers auf Basis von Dinatriumphosphat, zum Beispiel in einem Bereich von 0,001 mol/l bis 1 mol/l und bevorzugt von 0,005 mol/l bis 0,05 mol/l und insbesondere in einem Bereich um etwa 0,01 mol/l. Zum Beispiel können dem Puffergemisch noch Verbindungen aus der aus NaCl, Benzoesäure und weiteren gewöhnlich eingesetzten Pufferbestandteilen bestehenden Gruppe beigemengt werden.

Der erfindungsgemäßen Zusammensetzung können weitere Zusatzstoffe hinzugefügt werden, insbesondere Anti-Koagulationsmittel wie etwa EDTA, Heparin (z.B. Natriumheparinat), Citrat (z.B. Natriumcitrat), die einer Gerinnung des Blutes entgegenwirken. Desweiteren wirken sich andere übliche Plasmastabilisatoren wie Euxyl günstig aus. Aber auch andere herkömmlich verwendete Hilfs- und Zusatzstoffe wie etwa Reagenzien zur Bestimmung weiterer Blutbestandteile, verleihen der Zusammensetzung weitere nützliche Eigenschaften.

Die erfindungsgemäße Zusammensetzung liegt bevorzugt in fester Form, lyophylisierter Form oder als Lösung, beispielsweise in Wasser gelöst, vor. Die vorstehend gekennzeichnete Zusammensetzung der Erfindung wird erfindungsgemäß zur Stabilisierung von schwefelhaltigen Aminosäuren und/oder zur Hemmung der fortlaufenden Bildung von schwefelhaltigen Aminosäuren im Blut, z.B. im entnommenen Vollblut, im Blutplasma oder im Blutserum oder auch anderen Körperflüssigkeiten wie etwa Urin eingesetzt.

Gemäß der Erfindung kann jedoch auch ein Inhibitor allein, soweit dieser im Zusammenhang mit dem ersten Aspekt der Erfindung steht, für wenigstens eines der genannten an der metabolischen Homocysteinbildung beteiligten Enzyme zur Stabilisierung von schwefelhaltigen Aminosäuren und/oder zur Hemmung der fortlaufenden Bildung von schwefelhaltigen. Aminosäuren im Blut eingesetzt werden. Diesbezüglich wird auf die ohige Beschreibung der für den ersten Aspekt der Erfindung geeigneten Inhibitoren sowie der Kombination von mehreren dieser Inhibitoren verwiesen.

Die Verwendung entweder des Inhibitors allein oder der erfindungsgemäßen Zusammensetzung zielt auf die Stabilisierung der schwefelhaltigen Aminosäuren im Blut-Das in geeigneten Blutproben vorliegende Blut kann so nach dem Versetzen mit dem Inhibitor bzw, der erfindungsgemäßen Zusammensetzung für eine ausreichende Dauer aufbewahrt werden und braucht so erst mehrere Stunden oder Tage nach der Entnahme des Blutes untersucht zu werden. Wesentliche Verbesserungen der Präanalytik ergeben sich durch längere Lagerbeständigkeit, erweiterten Möglichkeiten der Analytik, insbesondere der zeitlichen Trennung von Blutentnahme und Messung der schwefelhaltigen Aminosäuren sowie des Transports der Blutproben, und der Gewährleistung von konstanten Werten für die gemessenen Aminosäuren auch nach Aufbewahrung bei Raumtemperatur über einen längeren Zeitraum.

Die Verwendung der Inhibitoren oder der Zusammensetzungen der Erfindung wird insbesondere für die Präanalytik und die Bestimmung von schwefelhaltigen Aminosäuren wie etwa Homocystein, Cystein, Cysteinylglycin, Glutathion und deren Derivate im Blut eingesetzt.

Bei der Verwendung der Säure enthaltenden erfindungsgemäßen Zusammensetzung wird die Konzentration der Säure bevorzugt so eingestellt, dass sie in einem Bereich von 2 bis 10 mg pro 1 ml zu untersuchendem Vollblut liegt.

Die Menge der einzelnen oder kombinierten Inhibitoren beziehungsweise der in der Zusammensetzung enthaltenden Inhibitoren wird geeigneterweise so eingestellt, dass pro 1 ml des zu untersuchenden Vollbluts eine Menge von 15 bis 210 µg und bevorzugt von 20 bis 100 *µ*g verwendet wird.

In dem erfindungsgemäßen Verfahren zur Stabilisierung von schwefelhaltigen Aminosäuren im Blut wird in Schritt (a) entweder der allein oder kombiniert verwendete Inhibitor oder die erfindungsgemäße Zusammensetzung gemäß dem beschriebenen erstem oder zweitem Aspekt in einer Blutsammelvorrichtung in einer vorbestimmten Menge vorgelegt. Die Menge an Inhibitor oder an der Zusammensetzung ist geeigneter Weise so bestimmt, dass die vorstehend genannten Konzentrationsbereiche für den Inhibitor und ggf. die Säure in Bezug auf die entnommene bzw. zu messende Blutprobe eingestellt werden.

Danach wird in Schritt (b) das entnommene Blut der Blutsammelvorrichtung zugeführt und unmittelbar die erhaltene Mischung vermischt. Je schneller diese Vermischung nach der Blutabnahme erfolgt, um so besser ist die Präanalytik und dadurch auch die anschließende Analytik der schwefelhaltigen Aminosäuren.

In Schritt (c) kann das in die Blutsammelvorrichtung eingebrachte und mit dem Inhibitor oder der Zusammensetzung der Erfindung vermischte Blut für eine bewünschte Zeit, über die die schwefelhaltigen Aminosäuren stabilisiert vorliegen bzw. die fortlaufende Bildung der schwefelhaltigen Aminosäuren gehemmt ist, aufbewahrt werden. Die erreichbaren Aufbewahrungszeiten hängen von dem verwendeten Inhibitor bzw. der Zusammensetzung ab und liegen gewöhnlich in einem Bereich von 48 bis 72 Stunden, können aber auch in einem Bereich von über 100 Stunden liegen.

Nach der Aufbewahrung kann gegebenenfalls in Schritt (d) die Analytik der schwefelhaltigen Aminosäuren und gegebenenfalls je nach Wunsch weiterer Blutbestandteile erfolgen. Die Bestimmung der gewünschten Aminosäuren erfolgt üblicher Weise im Blut selbst, z.B. aus dem Vollblut, dem Blutplasma und/oder dem Blutserum. Für die Analytik kann jedes im Stand der Technik bekannte Verfahren eingesetzt werden, wie zum Beispiel die Bestimmung mittels HPLC oder mittels eines Enzym-Immuno-Assays. Bevorzugt sind jeweils auf immunologischer Erfassung beruhende Bestimmungsverfahren.

Das erfindungsgemäße Verfahren kann ebenfalls so durchgeführt werden, dass nicht Inhibitor bzw. die den Inhibitor enthaltende Zusammensetzung in einer Blutsammelvorrichtung vorgelegt werden, sondern die verwendete Substanz erst nach dem Vorlegen der Blutprobe schnellstmöglich zu dieser hinzugegeben wird. So ist jede denkbare Art der Blutentnahme möglich, wenn das Blut sofort nach der Entnahme mit dem Inhibitor oder der erfindungsgemäßen Zusammensetzung in Kontakt gebracht und vermischt wird.

Je nachdem in welcher Reihenfolge das erfindungsgemäße Verfahren durchgeführt wird, kann es nötig sein unterschiedliche Blutsammelvorrichtungen einzusetzen.

Erfindungsgemäß umfasst eine Blutsammelvorrichtung entweder den allein verwendeten Inhibitor bzw. eine Kombination von Inhibitoren oder eine Zusammensetzung gemäß der Erfindung. Durch das Vorlegen des Inhibitors beziehungsweise der Zusammensetzung in der Blutsammelvorrichtung in einer festen, pulverförmigen, flüssigen oder lyophylisierten Form kann schnellstmöglich das entnommene Blut mit der vorgelegten Substanz vermischt werden. Die Blutsammelvorrichtung kann den einzelnen Inhibitor oder die Zusammensetzung ebenfalls in geträgerter Form umfassen.

Vorteilhafter Weise ist die Blutsammelvorrichtung mit einer Einrichtung ausgestaltet, die mit einer herkömmlichen Blutentnahmeeinrichtung wie etwa einer Injektionsnadel oder einer Braunüle direkt oder über einen Schlauch verbunden werden kann, um das entnommene Blut sofort mit der inhibierenden Substanz in Kontakt zu bringen. Deshalb ist es bevorzugt, dass die Blutsammelvorrichtung als eine Monovette ausgestaltet ist, welche den einzelnen Inhibitor beziehungsweise die erfindungsgemäße Zusammensetzung in gewünschter Form umfasst.

Die mit Blut und dem Inhibitor oder der Zusammensetzung befüllte Monovette kann verschlossen und ohne Kühlung unterschiedlich lange aufbewahrt und transportiert werden.

Anschließend wird die Erfindung durch die folgenden Beispiele detaillierter erläutert.

### Beispiele 1 bis 15 und Vergleichsbeispiele 1 bis 3

### Verwendetes Material und Verfahren

Folgende Methyltransferase- bzw. S-Adansylhomocysteinhydrolaseinhibitoren wurden getestet:
- 1: 5(4)-Aminoimidazol-4(5)-carboxamide (5(4)-AMCA)
- 2: 2'-Deoxyadenosin (DEOA)
- 3: 5-Deoxy-5-methyl-thioadenosin (5-DEMET)
- 4: 3-Deazaadenosin (3-DEAD)
- 5: Neplanocin A (NeplA)
- 6: 3-Deazaneplanocin A (3DA-NeplA)

Die Wirkung dieser Inhibitoren wurde in Kombination mit folgenden Säuren geprüft :
- 1: Ascorbinsäure (VitC)
- 2: Citronensäure (CS)
- 3: Citramalsäure (CMS)
- 4: Citraconsäure (COS)
- 5: Ethylendiamintetraessigsäure (EDTA)
- 6: Fumarsäure (FS)
- 7: Milchsäure (MS)
- 8: Oxalsäure (OS)
- 9: Weinsäure (WS)
die Zusammensetzungen der weiteren Reagenzien waren:
Mischung A: 5(4)-AMCA (5,2mg) und DEOA(3,2mg), gelöst in 500*µ*l Phosphatpuffer.
Zusammensetzung des Puffers für Mischung A: 0,01M Na₂ PO₄, 0,15M NaCl, 0,1% Benzoesäure, 0,1% Euxyl k100.
Alle andere Stoffe wurden in Wasser gelöst.

### Blutabnahme und Behandlung der Proben

In EDTA- oder Na-Heparin-Monovetten wurden für jedes Beispiel beziehungsweise Vergleichsbeispiel entsprechende Mengen an Stabilisatoren vorgelegt (s. Tabelle 1). Die Blutabnahme erfolgte in Monovetten mit und ohne Stabilosatoren. Für den Kontrollwert wurden die Monovetten innerhalb von 30 Minuten nach der Abnahme zentrifugiert.

### Ergebnisse

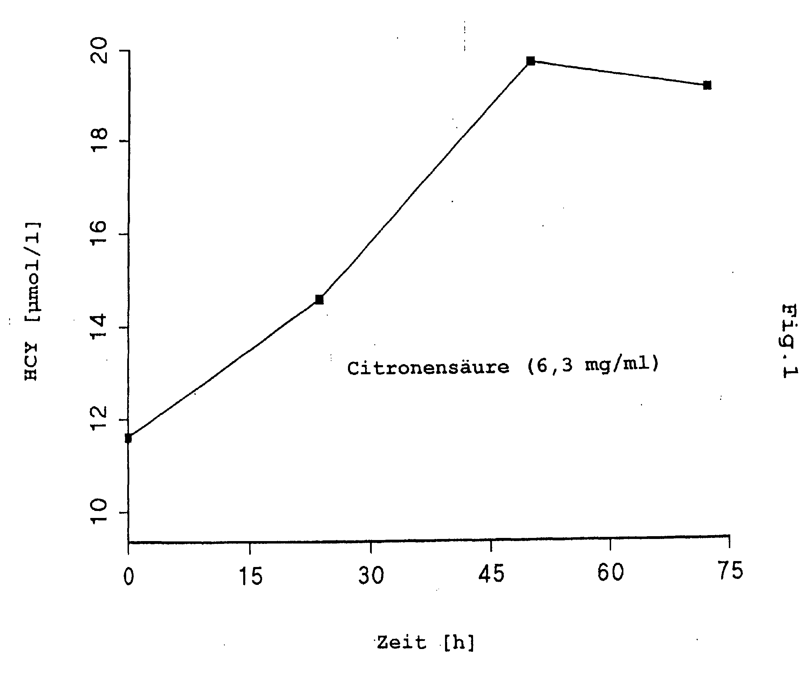

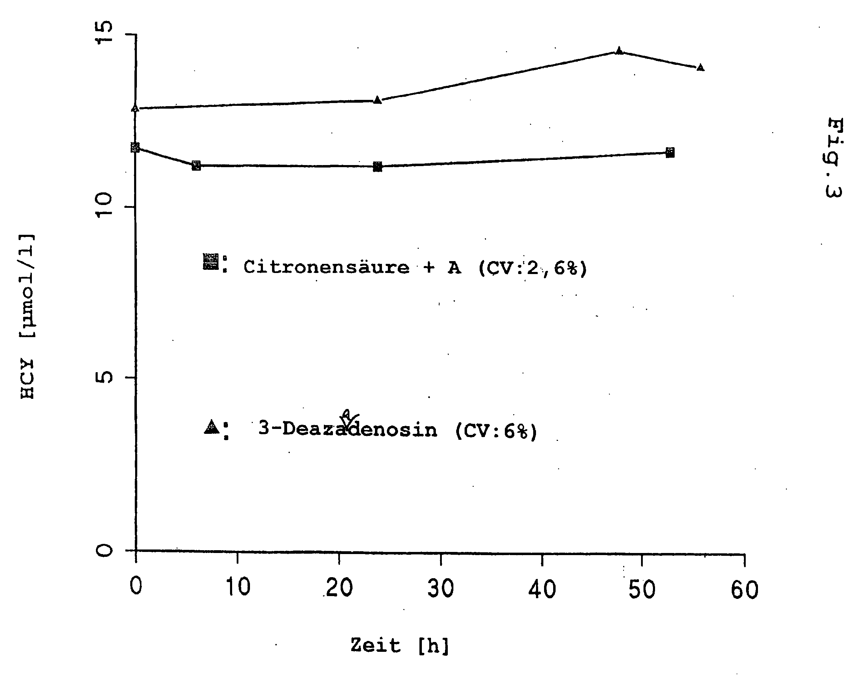

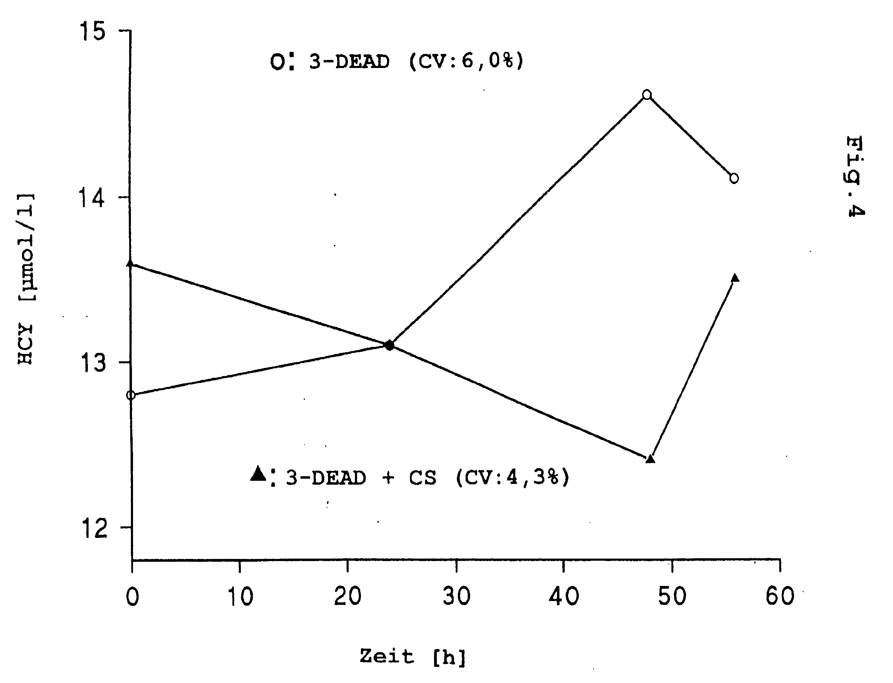

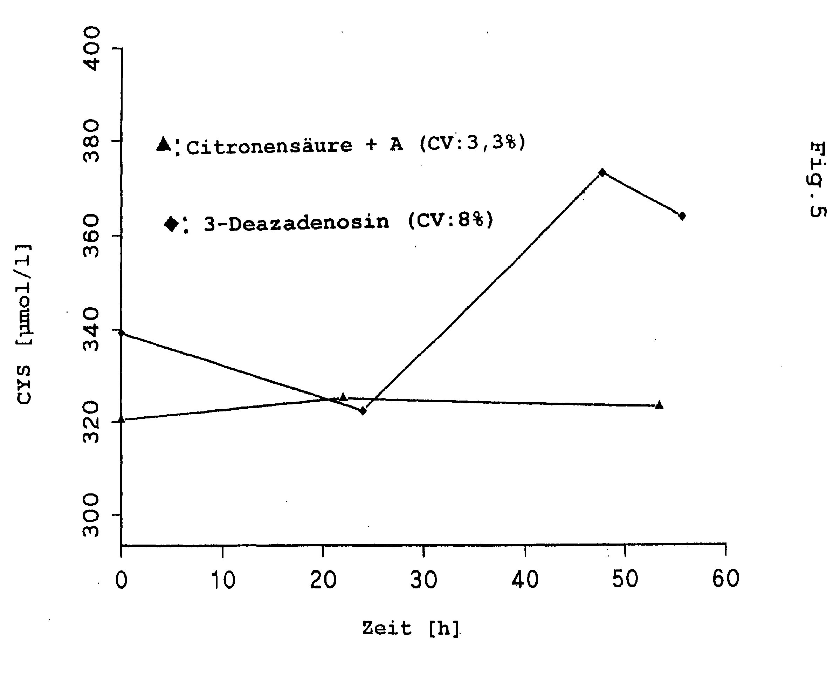

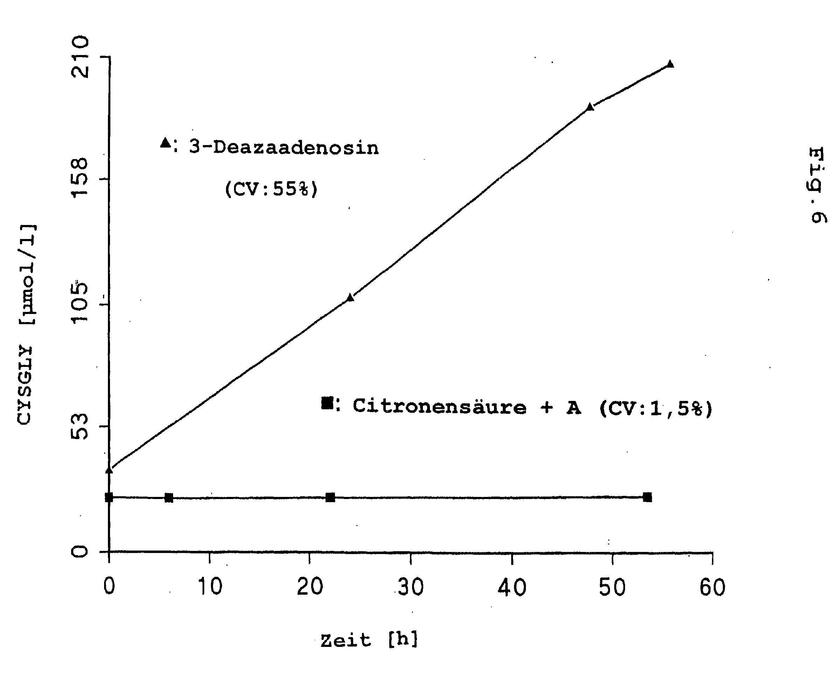

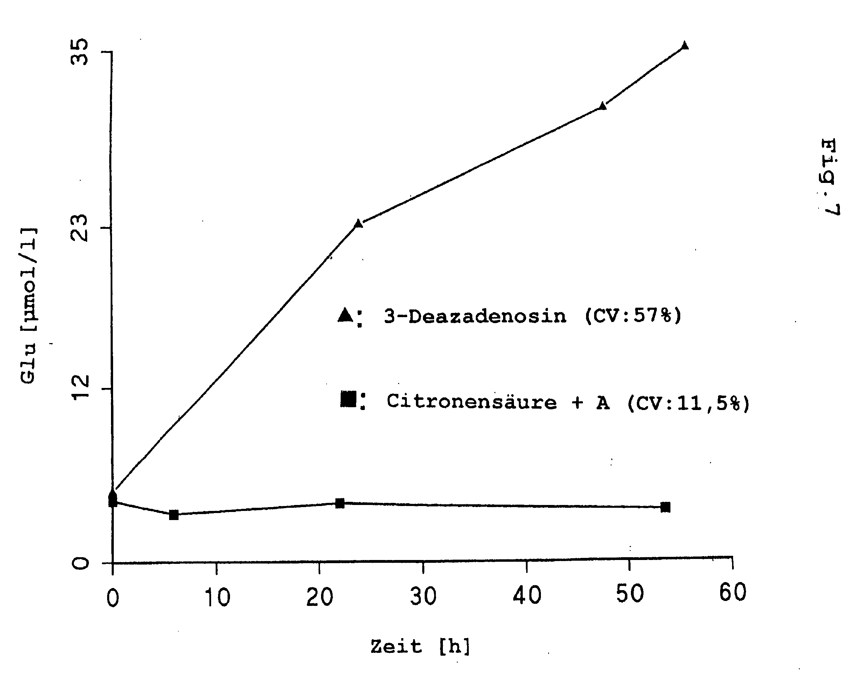

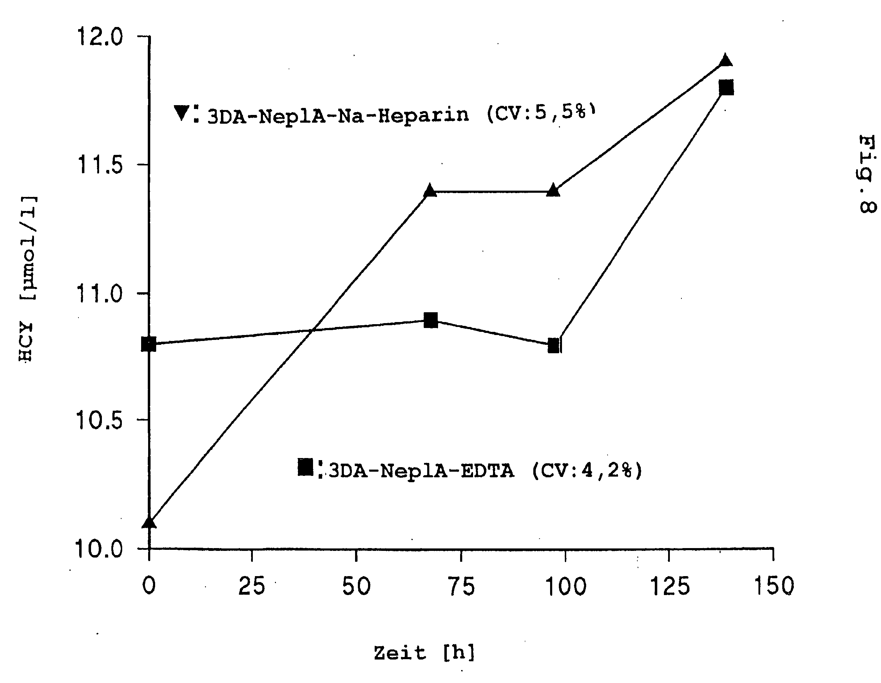

Die Ergebnisse für die Beispiele und Vergleichsbeispiele sind der Tabelle 1 und den Abbildungen 1 bis 8 zu entnehmen.

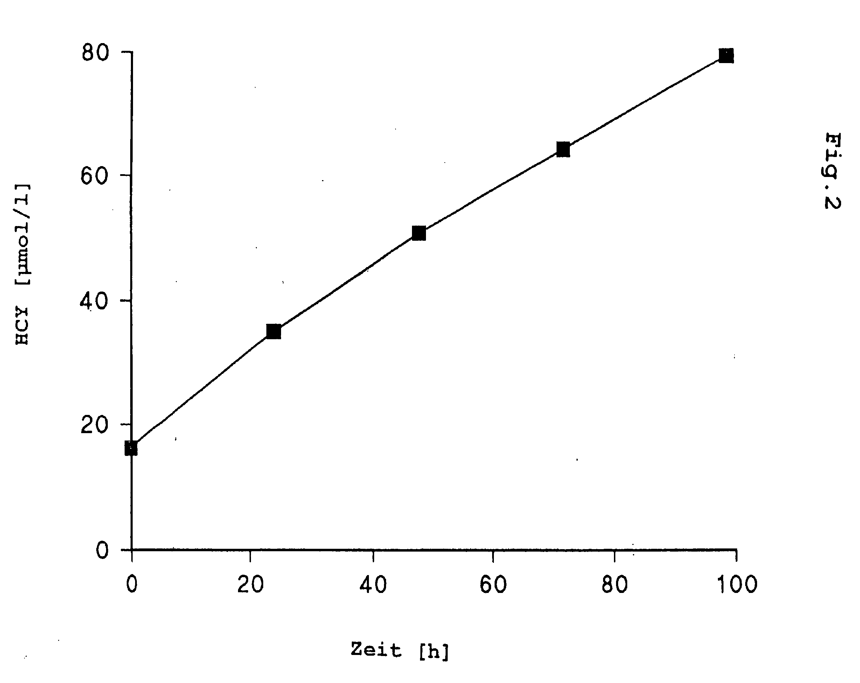

Fig. 1 und 2 zeigen, dass durch den Zusatz von Citronensäure und Na-EDTA allein in den Proben keine Stabilisierung der schwefelhaltigen Aminosäuren (HCY, CYS, CYSGLY, GLU) erfolgte. Furmarsäure und Ascorbinsäure konnten die Stabilisierung der HCY- und CYS-Konzentration über 40 Stunden gewährleisten, was aber vermutlich nur auf einem durch die Lyse der Erythrozyten hervorgerufenen Verdünnungseffekt beruhte.

Eine Mischung aus Säuren und Inhibitoren zeigte sich jedoch als potentieller Stabilisator für diese Aminosäuren (Tab.1). Die Mischung aus A und Citronensäure (Tab.1) erzielte die besten Ergebnisse hinsichtlich der gleichzeitigen Stabilisierung der Konzentration von HCY, CYS, CYSGLY und GLU über 50 Stunden (Tab.1).

Diese Ergebnisse verglichen mit den Daten, die durch eine Stabilisierung der Proben mit 3-Deazaadenosin erhalten wurden, sind in den Abbildungen 2 bis 8 dargestellt. Bei der ausschließlichen Verwendung von 5(4)-Aminoimidazol-4(5)-carboxamid oder 2'-Deoxyadenosin wurde keine signifikante Stabilisierung der schwefelhaltigen Aminosäuren erzielt. Dies deutet darauf hin, dass die Inhibitionswirkung dieser Stoffe in Anwesenheit von Protonenspendern gesteigert wird.

3DA-NeplA zeigt in diesen Versuchen die besten Ergebnisse (143 Stunden, Variationskoeffizient VK < 5 %) hinsichtlich der Stabilisierung von HCY im Vollblut.

**Tabelle 1**

| | | **Menge #** | **CYS-SD** | **HCY-SD** | **CYSGLY-SD** | **GLU-SD** |
|---|---|---|---|---|---|---|
| **Bsp.1** | CS + A' | 6,3 mg + 25 *µ*l | 72 h (VK:17%) | 103 h (VK:6.6%) | ( - ) | ( - ) |
| **Bp.2** | CS* + A | 6,3 mg + 25 *µ*l | 48 h (VK:3,5%) | 48 h (VK:2,1%) | ( - ) | ( - ) |
| **Bsp.3** | CS^{§} + A' | 6,3 mg + 25 *µ*l | 54 h (VK:3.3%) | 54 h (VK:2, 6%) | 54 h (VK:1,6%) | 54 h (VK:11% ) |
| **Bs.4** | CS^{$} + A' | 6,3 mg + 25 *µ*l | 73 h (Vk:17%) | 73 h (VK:7,2%) | 24 h (VK:35%) | ( - ) |
| **Vgl.-Bsp.1** | 3-DEAD | 0,107 mg | 56 h (VK:7,6) | 56 h (VK:6%) | ( - ) | ( - ) |
| **Bsp.5** | 3-DEAD + CS^{§} | 0,107 mg + 6,3 mg | 48 h (VK:3,6%) | 56 h (VK:4,3%) | ( - ) | ( - ) |
| **Bsp.6** | 3-DEAD + CS^{§} + A' | 0,107 mg + 6,3 mg + 25 µl | 24 h (VK.3,5%) | 24 h (Vk:2.1%) | ( - ) | ( - ) |
| **Vgl.-Bsp.2** | VitC | 6,3 mg | 73 h (VK:11%) | 73 h (VK:8%) | 73 h (VK:5%) | ( - ) |
| **Bsp.7** | VitC + A' | 6,3 mg + 25 µl | 72 h (VK:10%) | 73 h (VK:12%) | 73 h (VK:12%) | 73 h (VK:15% ) |
| **Bsp.8** | MS + A' | 2,8 mg + 25 µl | 72 h (VK:7,5%) | 48 h (VK:7,3) | ( - ) | 72 h (VK:6,7 %) |
| **Bp.9** | CMS + A' | 3,8 mg + 25 µl | 49 h (VK:3,8%) | 72 h (VK:7%) | 72 h (VK: 7, 5%) | ( - ) |
| **Bsp.10** | COS + A' | 3,8 mg + 25 µl | 72 h (VK:0,5%) | 72 h (VK:3,0%) | ( - ) | ( - ) |
| **Bsp.11** | 5-DEMET + A' | 5 mg + 25 µl | 49 h (VK:9%) | 49 h (VK:15%) | ( - ) | ( - ) |
| **Vgl.-Bs.3** | FS | 3,8 mg | 47 h (VK:4%) | 47 h (VK:8%) | ( - ) | ( - ) |
| **Bsp.12** | FS + A' | 6,3 mg + 25 µl | 44 h (VK:4,5%) | 44 h (VK:5%) | ( - ) | ( - ) |
| **Bsp.13** | NeplA | 60 µg | ( - ) | 70 h VK(4,5%) | ( - ) | ( - ) |
| **Bsp.14** | 3DA-NeplA | 30 µg | ( - ) | 143 h (VK4, 2%) | ( - ) | ( - ) |
| **Bsp.15** | OS + A' | 3,5 mg | 72 h (VK:3,5%) | 72 h (VK:4,5%) | ( - ) | ( - ) |

| | | | | | | |
|---|---|---|---|---|---|---|
| Anmerkungen zur Tab. 1: #: Menge pro ml Blut; SD: Stabilitätsdauer; CS^{§}: lyophilisiert; CS: Pulverform; CS*: Lösung; A': EDTA + A (5 (4) -AMCA + DEOA); A : A' ohne EDTA; ( - ) : keine Stabilisierung | | | | | | |

## Patentansprüche

1. Zusammensetzung zur Stabilisierung von schwefelhaltigen Aminosäuren und/oder zur Hemmung der fortlaufenden Bildung von schwefelhaltigen Aminosäuren im Blut, die folgendes enthält:
(i) ein Anti-Koagulationsmittel und/oder einen Plasmastabilisator; und
(ii-1) einen oder mehrere, nicht lediglich kompetitive(n) Inhibitor(en) der S-Adenosylhomocysteinhydrolase oder der Methyltransferase, die jeweils an der metabolischen Homocysteinbildung beteiligt sind, oder
(ii-2) eine Säure und wenigstens einen Inhibitor der S-Adenosylhomocysteinhydrolase oder der Methyltransferase, die jeweils an der metabolischen Homocysteinbildung beteiligt sind,
wobei (i) ein Zusatzstoff zu (ii-1) bzw. (ii-2) ist.

2. Zusammensetzung gemäß Anspruch 1, wobei der Inhibitor von (ii-1) oder (ii-2) nicht-ionisch ist und/oder so hydrophob ist, dass er zur Permeation einer Erythrocytenmembran in der Lage ist.

3. Zusammensetzung gemäß Anspruch 1 oder 2, wobei sie frei ist von Agenzien zur Lyse von Blutzellen.

4. Zusammensetzung gemäß Anspruch 1, wobei der Inhibitor von (ii-1) ein solcher ist, der zur Inaktivierung der S-Adenosylhomocysteinhydrolase unter Erschöpfung des Cofaktors NAD führt.

5. Zusammensetzung gemäß einem der vorangehenden Ansprüche,
wobei der Inhibitor von (ii-1) aus der Gruppe ausgewählt ist, die aus Neplanocin A, 3-Deazaneplanocin A, 9-(*trans*-2'-*trans*-3'-dihydroxycylopent-4'-enyl)-adenosin (DHCeA), 3-Deaza-DHCeA, 9- (*trans*-2'-*trans*-3'-dihydxoxycylopentanyl)-adenosin (DHCaA), 3-Deza-DHCaA, β-4'-Methyl-DHCaA, β-4'-Vinyl-DHCaA, (Z- oder E-)4', 5'-Didehydro-5'-deoxy-5'-fluoro-adenosin (Z-DDFA oder E-DDFA) und 5'-Deoxy-5'-difluoro-adenosin (DFA) sowie den inhibitorischen Derivaten oder Analoga der genannten verbindungen besteht; oder
wobei der Inhibitor von (ii-2) aus der Gruppe ausgewählt wird, die aus 2'-Deoxyadenosin (DEOA), 5(4)-Aminoimidazol-4(5)-carboxamid (5 (4) -AMCA), 9 (s) - (2,3-Dihydroxypropyl) -adenosin ((S)-DHPA), (R,S)-3-Adenin-9-yl-2-hydroxypropansäure ((R,S)-AHPA), Adenin-dialdehyd, 3-Deazaadenosin, 5-Deoxy-5-methyl-thioadenosin, 5-Deoxyadenosin (Aristeromycin), Neplanocin A, 3-Deazaneplanocin A, 9-(*trans*-2'-,*trans*-3'-dihydroxycylopent-4'-enyl)-adenosin (DHCeA), 3-Deaza-DHCeA, 9-(*trans*-2'-,*trans*-3'-dihydroxycyclopentanyl)-adenosin (DHCaA), 3-Deza-DHCaA, β-4' - Methyl-DHCaA, β-4'-Vinyl-DHCaA, (Z- oder E-) 4', 5'-Didehydro-5'-deoxy-5'-fluoro-adenosin (Z-DDFA oder E-DDFA) und 5'-Deoxy-5'-difluoro-adenosin (DFA) sowie den inhibitorischen Derivaten oder Analoga der genannten Verbindungen besteht.

6. Zusammensetzung gemäß Anspruch 1, wobei die Säure von (ii-2) aus der Gruppe ausgewählt ist, die aus Ascorbinsäure, Citronensäure, Citramalsäure, Citraconsäure, Fumarsäure, Milchsäure, Oxalsäure und Weinsäure besteht.

7. Zusammensetzung gemäß Anspruch 1 oder 6, wobei die Säure von (ii-2) in Pulverform oder in lyophilisierter Form vorliegt.

8. Zusammensetzung gemäß einem der vorangehenden Ansprüche, umfassend eine Kombination verschiedener Inhibitoren , wobei die Kombination aus einem oder mehreren kompetitiven Inhibitor(en) oder einem oder mehreren irreversiblen Inhibitor(en) gebildet ist.

9. Zusammensetzung gemäß Anspruch 8; wobei 2'-Deoxyadenosin mit 5(4)-Aminoimidazol -4(5)-carboxamid kombiniert ist.

10. Zusammensetzung gemäß einem der vorhergehenden Ansprüche, die zusätzlich einen Puffer enthält.

11. Verwendung eines Inhibitors der S-Adenosylhomocysteinhydrolase oder der Methyltransferase zur Stabilisierung von schwefelhaltigen Aminosäuren und/oder zur Hemmung der fortlaufenden Bildung von schwefelhaltigen Aminosäuren im entnommenen Blut für die Präanalytik, wobei als Inhibitor ein nicht lediglich kompetitiver Inhibitor der S-Adenosylhomocysteinhydrolase oder der Methyltransferase, die jeweils an der metabolischen Homocysteinbildung beteiligt sind, verwendet wird.

12. Verwendung gemäß Anspruch 11, wobei der verwendete Inhibitor nicht-ionisch ist und/oder so hydrophob ist, dass er zur Permeation einer Erythrocytenmembran in der Lage ist.

13. Verwendung gemäß Anspruch 11 oder 12, wobei der Inhibitor ohne Agenzien zur Lyse von Blutzellen verwendet wird.

14. Verwendung gemäß einem der Ansprüche 11 bis 13, wobei ein Inhibitor der S-Adenosylhomocysteinhydrolase verwendet wird.

15. Verwendung gemäß Anspruch 14, wobei ein Inhibitor verwendet wird, der zur Inaktivierung der S-Adenosylhomocysteinhydrolase unter Erschöpfung des Cofaktors NAD führt.

16. Verwendung gemäß Anspruch 11, wobei der verwendete Inhibitor aus der Gruppe ausgewählt wird, die aus Neplanocin A, 3-Deazaneplanocin A, 9-(*trans*-2'-*trans*-3'-dihydroxycylopent-4'-enyl)-adenosin (DHCeA), 3-Deaza-DHCeA, 9-(*trans*-2'-,*trans-*3'-dihydroxycylopentanyl)-adenosin (DHCaA), 3-Deza-DHCaA, β-4'-Methyl-DHCaA, β-4'-Vinyl-DHCaA, Z- oder E-4',5'-Didehydro-5', deoxy-5'-fluoro-adenosin (Z-DDFA oder E-DDFA) und 5' -Deoxy-5' - difluoro-adenosin (DFA) sowie den inhibitorischen Derivaten oder Analoga der genannten Verbindungen besteht.

17. Verwendung der Zusammensetzung gemäß einem der Ansprüche 1 bis 10 zur Stabilisierung von schwefelhaltigen Aminosäuren und/oder zur Hemmung der fortlaufenden Bildung von schwefelhaltigen Aminosäuren im Blut.

18. Verwendung gemäß Anspruch 17, wobei die schwefelhaltigen Aminosäuren aus Homocystein, Cystein, Cysteinylglycin, Glutathion und deren Derivaten ausgewählt sind.

19. Verwendung gemäß einem der Ansprüche 11 bis 18 zur stabilisierung und/oder Aufbewahrung von Blutproben.

20. Verwendung gemäß einem der Ansprüche 17 bis 19, wobei im Fall der Verwendung einer Säure die Säure in einem Konzentrationsbereich von 2 bis 10 mg pro 1 ml des zu untersuchenden Blutes liegt.

21. Verwendung gemäß einem der Ansprüche 11 bis 20, wobei der Inhibitor in einem Konzentrationsbereich von 15 bis 210 *µ*g pro 1 ml des zu untersuchenden Blutes liegt.

22. Verwendung gemäß einem der Ansprüche 11 bis 21, wobei gleichzeitig oder nach der Verwendung des Inhibitors ein Test zur Bestimmung mindestens einer schwefelhaltigen Aminosäure und gegebenenfalls eines weiteren Blutbestandteile durchgeführt wird.

23. Verwendung gemäß einem der Ansprüche 17 bis 22 für die Präanalytik.

24. VA-rfahren zur Stabilisierung von schwefelhaltigen Aminosäuren und/oder zur Hemmung der fortlaufenden Bildung von schwefelhaltigen Aminosäuren in entnommenem Blut, wobei das Blut nach der Entnahme mit einem wie in einem der Ansprüche 11 bis 16 definierten Inhibitor oder mit einer Zusammensetzung gemäß einem der Ansprüche 1 bis 10 vermischt wird.

25. Verfahren gemäß Anspruch 24, wobei gegebenenfalls der Gehalt an schwefelhaltigen Aminosäuren im Blut und/oder andere Blutbestandteile bestimmt, werden, das die folgenden Schritte umfasst:
(a) Einbringen einer Zusammensetzung gemäß einem der Ansprüche 1 bis 10 in einer Plutsammelvorrichtung;
(b) Einbringen von Blut in die Blutsammelvorrichtung;
gegebenenfalls (c) Aufbewahrung des in die Blutsammelvorrichtung eingehrachten Blutes für eine bestimmte Zeit, über die die schwefelhaltigen Aminosäuren stabilisiert vorliegen und/oder die fortlaufende Bildung der schwefelhaltigen Aminosäuren gehemmt ist; und
gegebenenfalls (d) Bestimmung des Gehalts der gewünschten schwefelhaltigen Aminosäuren und gegebenenfalls weiterer Blutbestandteile in der Blutprobe.

26. Verfahren gemäß Anspruch 25, wobei die Schritte (a) und (b) vertauscht sind.

27. Verfahren gemäß einem der Ansprüche 24 bis 26, wobei der Gehalt der gewünschten schwefelhaltigen Aminosäuren mittels HPLC bestimmt wird.

28. Verfahren gemäß einem der Ansprüche 24 bis 26, wobei der Gehalt der gewünschten schwefelhaltigen Aminosäuren mittels eines Enzym-Immuno-Assays bestimmt wird.

29. Blutsammelvorrichtung, die eine Zusammensetzung gemäß einem der Ansprüche 1 bis 10 umfasst.

30. Blutsammelvorrichtung gemäß Anspruch 29, die eine mit einer herkömmlichen Blutentnahmecinrichtung verbindbar ausgestaltete Einrichtung umfasst.

31. Blutsammelvorrichtung gemäß Anspruch 29 oder 30, die als eine Monovette ausgestaltet ist.

## Claims

1. A composition for the stabilization of sulphur-containing amino acids and/or for the inhibition of the continuous formation of sulphur-containing amino acids in blood, which composition contains the following:
(i) an anti-coagulant and/or a plasma stabilizer; and
(ii-1) one or more, not merely competitive inhibitor(s) of S-adenosylhomocysteine hydrolase or methyl transferase, which are respectively involved in metabolic formation of homocysteine, or
(ii-2) and acid and at least one inhibitor of S-adenosylhomocysteine hydrolase or methyl transferase, which are respectively involved in metabolic formation of homocysteine;
wherein (i) is a substance additional to (ii-1) or (ii-2).

2. The composition according to claim 1, wherein the inhibitor (ii-1) or (ii-2) is non-ionic and/or is such hydrophobic as to be capable of permeating an erythrocyte membrane.

3. The composition according to claim 1 or 2, wherein it is free of agents for the lyses of blood cells.

4. The composition according to claim 1, wherein the inhibitor (ii-1) is one which leads to the inactivation of S-adenosylhomocysteine hydrolase with depletion of the co-factor NAD.

5. The composition according to one of the preceding claims, wherein the inhibitor of (ii-1) is selected from the group consisting of neplanocin A, 3-deazaneplanocin A, 9-(*trans-2', trans*-3'-dihydroxycylopent-4'-enyl)-adenosin (DHCeA), 3-deaza-DHCeA, 9-(*trans*-*2*'-,*trans*-3'-dihydroxycylopentanyl)-adenosin (DHCaA), 3-deza-DHCaA, β-4'-methyl-DHCaA, β-4'-vinyl-DHCaA, (Z- or E-)4',5'-didehydro-5'-deoxy-5'-fluoro-adenosin (Z-DDFA or E-DDFA) and 5'-deoxy-5'-difluoro-adenosin (DFA) as well as the inhibitory derivatives or analogues of the mentioned compositions; or
wherein the inhibitor (ii-2) is selected from the group consisting of 2'-deoxyadenosin (DEOA), 5(4)-aminoimidazol-4(5)-carboxamid (5(4)-AMCA), 9(S)-(2,3-dihydroxypropyl)-adenosin ((S)-DHPA), (R,S)-3-adenin-9-yl-2-hydroxypropane acid ((R,S)-AHPA), adenin-dialdehyd, 5-deazaadenosin, 5-deoxy-5-methyl-thioadenosin, 5-deoxyadenosin (aristeromycin), neplanocin A, 3-deazaneplanocin A, *9-(trans-*2'*-,trans-*3'-dihydroxycylopent-4'-enyl)-adenosin (DHCeA), 3-deaza-DHCeA, 9-(*trans*-2'-,*trans*-3'-dihydroxycylopentanyl)-adenosin (DHCaA), 3-deza-DHCaA, β-4'-methyl-DHCaA, β-4'-vinyl-DHCaA, (Z or E-)4', 5'-didehydro-5'-deoxy-5'-fluoro-adenosin (Z-DDFA or E-DDFA) and 5'-deoxy-5'-difluoro-adenosin (DFA) as well as the inhibitory derivatives or analogues of the mentioned compositions.

6. The composition according to claim 1, wherein the acid of (ii-2) is selected from the group consisting of ascorbic acid, citric acid, citramalic acid, citraconic acid, fumaric acid, lactic acid, oxalic acid and tartaric acid.

7. The composition according to claim 1 or 6, wherein the acid of (ii-2) is present in powdered form or in lyophilized form.

8. The composition according to one of the preceding claims, comprising a combination of different inhibitors, wherein the combination is formed of one or more competitive inhibitor(s) or one or more irreversible inhibitor(s).

9. The composition according to claim 8, wherein 2'-deoxyadenosin is combined with 5(4)-aminoimidazol-4(5)-carboxamid.

10. The composition according to one of the preceding claims, further containing a buffer.

11. A use of an inhibitor of S-adenosylhomocysteine hydrolase or methyl transferase for the stabilization of sulphur-containing amino acids and/or for the inhibition or the continuous formation of sulphur-containing amino acids in blood, wherein a not merely competitive inhibitor of S-adenosylhomocysteine hydrolase or methyl transferase, which are involved in the metabolic formation of homocysteine, is used as inhibitor.

12. The use according to claim 11, wherein the used inhibitor is non-ionic and/or is such hydrophobic so as to be capable of permeating an erythrocyte membrane.

13. The use according to claim 11 or 12, wherein the inhibitor is used without agents for the lyses of blood cells.

14. The use according to one of claims 11 to 13, wherein an inhibitor of S-adenosylhomocysteine hydrolase is used.

15. The use according to claim 14, wherein an inhibitor is used which leads to the inactivation of the S-adenosylhomocysteine hydrolase with a depletion of the co-factor NAD.

16. The use according to claim 11, wherein the used inhibitor is selected from the group consisting of neplanocin A, 3-deazaneplanocin A, 9-(*trans*-2'-, *trans*-3'-dihydroxycylopent-4'-enyl)-adenosin (DHCeA), 3-deaza-DHCeA, 9-(*trans-*2'*-,trans-*3'-dihydroxycylopentanyl)-adenosin (DHCaA), 3-deaza-DHCaA, β-4'-methyl-DHCaA, β-4'-vinyl-DHCaA, Z- or E-4', 5'-didehydro-5'-deoxy-5'-fluoro-adenosin (Z-DDFA or E-DDFA) and 5'-deoxy-5'-difluoro-adenosin (DFA) as well as the inhibitory derivatives or analogues of the mentioned compositions.

17. The use of the composition according to one of claims 1 to 10 for the stabilization of sulphur-containing amino acids and/or for the inhibition of the continuous formation of sulphur-containing amino acids in blood.

18. The use according to claim 17, wherein the sulphur-containing amino acids are selected among homocysteine, cysteine, cysteinyl glycine, glutathione and their derivatives.

19. The use according to one of claims 11 to 18 for the stabilization and/or storage of blood samples.

20. The use according to one of claims 17 to 19, wherein, in the case of using an acid, the acid is in a concentration range of from 2 to 10 mg per 1 ml of the blood to be tested.

21. The use according to one of claims 11 to 20, wherein the inhibitor lies in a concentration range of from 15 to 210 µg per 1 ml of the blood to be tested.

22. The use according to one of claims 11 to 21, wherein, concurrently with or after the use of the inhibitor, a test is carried out for the determination of at least one sulphur-containing amino acid and optionally a further blood component.

23. The use according to one of claims 17 to 22 for preparative analytical medicine.

24. A process for the stabilization of sulphur-containing amino acids and/or for the inhibition of the continuous formation of sulphur-containing amino acids in blood, wherein the blood, after its withdrawal, is mixed with an inhibitor as defined in one of claims 11 to 16, or with a composition according to one of claims 1 to 10.

25. The process according to claim 24, wherein optionally the amount of sulphur-containing amino acids in blood and/or other blood components are determined by comprising the following steps:
(a) placing a composition according to one of claims 1 to 10 in a blood collecting device;
(b) placing blood in the blood collecting device; optionally (c) storing the blood placed in the blood collecting device for a predetermined period in which the sulphur-containing amino acids are present in a stabilized manner and/or the continuous formation of the sulphur-containing amino acids is inhibited; and
optionally (d) determining the amount of the desired sulphur-containing amino acid and optionally further blood components in the blood sample.

26. The process according to claim 25, wherein the steps (a) and (b) are reversed.

27. The process according to one of claims 24 to 26, wherein the amount of the desired sulphur-containing amino acid is determined by means of HPLC.

28. The process according to one of claims 24 to 26, wherein the amount of the desired sulphur-containing amino acid is determined by means of an enzyme immunoassay.

29. Blood collecting device comprising a composition according to one of claims 1 to 10.

30. The blood collecting device according to claim 29, comprising a device which is capable of being connected with a conventional blood withdrawal device.

31. The blood collecting device according to claim 29 or 30, which is designed in the form of a monovette.

## Revendications

1. Composition pour stabiliser des acides aminés contenant du soufre et / ou freiner la formation continue des acides aminés contenant du soufre dans le sang, comportant le suivant :
(i) un moyen anticoagulant et / ou un stabilisateur de plasma; e t
(ii-1) un ou plusieurs inhibiteur (s), pas seulement compétitif(s), de la S-adénosyle homocystéine hydrolase ou de la méthyltransférase, qui participent respectivement à la formation métabolique de l'homocystéine,
ou
(ii-2) un acide et au moins un inhibiteur de la S-adénosyle homocystéine hydrolase ou de la méthyltransférase, qui participent respectivement à la formation métabolique de l'homocystéine ,
où (i) est un additif à (ii-1) respectivement (ii-2).

2. Composition selon la revendication 1, où l'inhibiteur de (ii-1) ou (ii-2) est non ionique et / ou si hydrophobe qu'il permet une perméation d'une membrane d'érythrocytes.

3. Composition selon les revendications 1 ou 2, où ladite composition est libre d'agents pour la lyse des cellules du sang.

4. Composition selon la revendication 1, où l'inhibiteur de (ii-1) est un inhibiteur, qui conduit à l'inactivation de la S-adénosyle homocystéine hydrolase en déplétant le cofacteur NAD .

5. Composition selon une des revendications précédentes, où l'inhibiteur de (ii -1) est choisi du groupe qui est composé de néoplanocine A, 3°- déazanéplanocine A, 9-(trans-2',trans-3'-dihydroxycylopent-4'-ényle)-adénosine (DHCaA), 3 - déaza - DHCaA, 9-(trans-2'-,trans-3'-dihydroxycylopentanyl)-adenosin (DHCaA), 3-Deza-DHCaA, β- 4'-méthyle - DHCaA, β - 4'-vinyle -DHCaA, (z - ou E') 4', 5' - didéhydro - 5'- déoxy - 5' - fluoro - adénosine (Z - DDFA ou E-DDFA) et 5'-déoxy - 5' - difluoro - adénosine (DFA) ainsi que des dérivés inhibiteurs ou des analogues des composés cités; ou
où l'inhibiteur de (ii-2) est choisi du groupe qui est composé de 2'-déoxyadénosine (DEOA), 5 (4) - aminoimidazol- 4 (5) -carboxamide (5 (4) - AMCA), 9 (S) - (2,3 - dihydroxypropyle) -adénosine ((S) - DHPA), l'acide hydroxy propane (R,S)-3-adénine - 9 - yl- 2 ((R,S} AHPA), adénine - dialdéhyde, 3 - déazaadénosine, 5 - déoxy - 5 - méthyle -thieadénosine, 5 - déoxyadénosine (aristéromycine), néplanocine A, 3 -deazaneplanocine A, 9 - (trans⁻2'-, trans-3' - dihydroxycylopent - 4' - ényle)- adénosine (DHCeA), 3- deaza-DHCeA, 9⁻(trans-2'-, trans - 3' -dihydroxycylopentanyle)-adénosine (DHCaA), 3-deza-DHCaA, β - 4' -méthyle - DHCaA, β - 4'- vinyle -DHCaA, (Z - ou E-) 4' , 5'- didéhydro-5' - déoxy -5'- fluoro - adénosine (Z-DDFA ou E-DDFA) et 5'- déoxy - 5' -difluoro - adénosine (DFA) ainsi que des dérivés inhibiteurs ou des analogues des composés cités.

6. Composition selon la revendication 1, où l'acide de (ii - 2) est choisi du groupe qui est composé de l'acide ascorbique, de l'acide citrique, de l'acide citramalique, de l'acide citraconique, de l'acide fumarique, de l'acide lactique, de l'acide oxalique et de l'acide tartrique.

7. Composition selon les revendications 1 ou 6, où l'acide de (ii-2) est présent sous une forme en poudre ou sous une forme lyophilisée.

8. Composition selon une des revendications précédentes, comportant une combinaison de différents inhibiteurs, où la combinaison est formée par un ou plusieurs inhibiteur (s) compétitif (s) ou par un ou plusieurs inhibiteur (s) irréversible (s).

9. Composition selon la revendication 8; où le 2'-déoxyadénosine est combiné avec le 5(4)-aminoimidazol -4 (5)-carboxamide.

10. Composition selon une des revendications précédentes
, qui comporte en plus un tampon.

11. Utilisation d'un inhibiteur de la S - adénosyl - homocystéine hydrolase ou de la méthyle - transférase pour la stabilisation d'acides aminés contenant du soufre et / ou pour inhiber la formation continue d'acides aminés contenant du soufre dans le sang prélevé pour la préanalytique, où l'on utilise en tant qu'inhibiteur un inhibiteur qui n'est pas seulement compétitif de la S - adénosyl homocystéine - hydrolase ou de la méthyle transférase, qui participent respectivement à la formation métabolique de homocystéine .

12. Utilisation selon la revendication 11, où l'inhibiteur utilisé est non-ionique et / ou si hydrophobe, qu'il permet une perméation d'une membrane d'érythrocyte.

13. Utilisation selon la revendication 11 ou 12, ou l'inhibiteur est utilisé sans agents pour la lyse de cellules sanguines .

14. Utilisation selon la revendication 11 à 13., ou est utilisé l'inhibiteur de la S - adénosyl homocystéine - hydrolase.

15. Utilisation selon la revendication 14, où est utilisé un inhibiteur , qui conduit à l'inactivation de la S - adénosyl homocystéine - hydrolase en déplétant le cofacteur NAD

16. Utilisation selon la revendication 11, où l'inhibiteur utilisé est choisi du groupe qui est composé de 2'-déoxyadénosine (DEOA), 5 (4) - aminoimidazol- 4 (5) - carboxamide (5 (4) -AMCA), 9 (S) - (2,3 - dihydroxypropyle) -adénosine ((S) - DHPA), l'acide hydroxy propane (R,S)-3-adénine - 9 - y1- 2 ((R,S}AHPA), adénine - dialdéhyde, 3 - déazaadénosine, 5 - déoxy - 5 - méthyle -thieadénosine, 5 - déoxyadénosine (aristéromycine), néplanocine A, 3 -deazaneplanocine A, 9 - ( trans⁻2'-, trans⁻3' - dihydroxycylopent - 4' - ényle)- adénosine (DHCeA), 3- deaza-DHCeA, 9'(trans-2'-, trans - 3' -dihydroxycylopentanyle)-adénosine (DHCaA), 3-deza-DHCaA, β- 4' -méthyle - DHCaA, β - 4'- vinyle -DHCaA, (Z - ou E-) 4' , 5'- didéhydro-5' - déoxy -5'- fluoro - adénosine (Z-DDFA ou E-DDFA) et 5'- déoxy - 5' -difluoro - adénosine (DFA) ainsi que des dérivés inhibiteurs ou des analogues des composés cités.

17. Utilisation de la composition selon une des revendications 1 à 10, pour la stabilisation d'acides aminés contenant du soufre et / ou pour l'inhibition de la formation continue d'acides aminés, contenant du soufre, dans le sang.

18. Utilisation selon la revendication 17, où les acides aminés contenant du soufre sont choisis parmi homocystéine, la cystéine, la cystéinyle - glycine, le gluthanione et de leurs dérivés.

19. Utilisation selon une des revendications 11 à 18 pour la stabilisation et / ou la conservation d'échantillons de sang.

20. Utilisation selon une des revendications 17 à 19, où, dans le cas de l'utilisation d'un acide, le degré d'acidité est situé dans une zone de concentration de 2 à 10 mg par 1 ml de sang à analyser.

21. Utilisation selon une des revendications 11 à 20, où l'inhibiteur est situé dans une zone de concentration de 15 à 210 *µ*g par 1 ml de sang à analyser.

22. Utilisation selon une des revendications 11 à 21, où on réalise mutuellement ou après l'utilisation de l'inhibiteur un test pour la détermination d'au moins un acide aminé contenant du soufre et éventuellement un composant supplémentaire du sang.

23. Utilisation selon une des revendications 17 à 22 pour la préanalytique .

24. Procédé pour la stabilisation d'acides aminés contenant du soufre et / ou pour inhiber la formation continue d'acides aminés contenant du soufre dans le sang prélevé pour la préanalytique, où le sang est mélangé après le prélèvement avec un inhibiteur défini dans une des revendications 11 à 16 ou avec une composition selon une des revendications 1 à 10.

25. Procédé selon la revendication 24, où on détermine éventuellement la teneur en acides aminés contenant du soufre dans le sang et / ou d'autres composants du sang, lequel procédé comporte les étapes suivantes :
(a) introduction d'une composition selon une des revendications 1 à 10 dans un dispositif de collecte de sang;
(b) introduction de sang dans un dispositif de collecte de sang; éventuellement (c) conservation du sang introduit dans le dispositif de collecte de sang pour un temps déterminé , pendant lequel les acides aminés contenant du soufre sont présents de façon stabilisée et / ou la formation continue des acides aminés contenant du soufre est inhibée ; et
éventuellement (d) détermination de la teneur des acides aminés contenant du soufre souhaités et éventuellement d'autres composant de sang dans l'échantillon sanguin.

26. Procédé selon la revendication 25, où les étapes (a) et (b) sont échangés.

27. Utilisation selon une des revendications 24 à 26, où la teneur des acides aminés contenant du soufre souhaités est déterminé au moyen de l'HPLC .

28. Utilisation selon une des revendications 24 à 26, où la teneur des acides aminés contenant du soufre souhaités est déterminée au moyen d'une analyse immuno-enzymatique.

29. Dispositif de collecte de sang, qui contient une composition selon une des revendications 1 à 10.

30. Dispositif de collecte de sang selon la revendication 29, qui comporte un dispositif qui est réalisé de façon à pouvoir être relié avec un dispositif de collecte de sang habituel.

31. Dispositif de collecte de sang selon les revendications 29 ou 30, qui est réalisé comme monovette.
